# EUROPEAN PATENT APPLICATION

(11) **EP 3 135 182 A1**
(43) Date of publication of application: **01.03.2017**
(21) Application number: 15881153.9
(22) Date of filing: 17.09.2015
(51) Int. Cl.: A61B 1/00, G02B 23/24

(54) **ENDOSCOPE BENDING SECTION AND ENDOSCOPE**

(30) Priority: 03.02.2015 JP 2015019689
(71) Applicant: Olympus Corporation, Tokyo 192-8507 (JP)
(72) Inventor: NAKADE Sho, Tokyo 192-8507 (JP)
(74) Representative: Schicker, Silvia
(86) International application number: PCT/JP2015/076517
(87) International publication number: WO 2016/125336

(57) **Abstract**

An endoscope bending portion 7 includes a tubular member 40 provided with a treatment instrument channel 26 and a string guide 49 configured to hold wires 47 and 48, an interval t1 of a plurality of first slots 41 formed in a first tubular region 40a on a distal end side is made to be smaller than an interval t2 of a plurality of second slots 42 formed in a second region 40b at an intermediate portion, and a third interval t3 of a plurality of third slots 43 formed in a third tubular region 40c is also made to be smaller than the interval t2 of the plurality of second slots 42, and a dimensional ratio between a width w1 and the interval t1 of the first slots 41, a dimensional ratio between a width w2 and the interval t2 of the second slots 42 and a dimensional ratio between a width w3 and the interval t3 of the third slots 43 are equalized.

## Description

### Technical Field

The present invention relates to an endoscope bending portion provided with a bending portion which is bent through hand operation and an endoscope.

### Background Art

In recent years, devices inserted into an elongated tube, for example, endoscopes in particular, have been widely used in a medical field and an industrial field.

Especially, endoscopes used in the medical field can be used to observe organs in a body cavity by inserting an elongated insertion portion into the body cavity which is a subject or perform various types of treatment by using, if necessary, a treatment instrument inserted into a treatment instrument insertion channel provided for the endoscope.

It is common knowledge that such conventional endoscopes adopt a configuration in which the insertion portion is provided with a bending portion which is freely bendable to improve insertability into a subject.

Among conventional endoscopes, various endoscopes have appeared such as endoscopes with a bending portion provided in the insertion portion including a plurality of metallic bending pieces coupled together via rivets or the like, and in recent years, endoscopes with a bending portion made up of an ultra-elastic pipe in which slots are formed through a machining process as disclosed, for example, in Japanese Patent No. 5444516.

Such conventional bending portions are bent via a wire which is pulled or slackened through hand operation using operation members such as an operation lever and an operation knob provided at the operation portion.

However, the endoscope disclosed in Japanese Patent No. 5444516 has a problem that while the bending portion is bent, if a treatment instrument is inserted into a treatment instrument channel in the bending portion, a specific region of the treatment instrument channel is pressed against a string guide that guides a wire for operating the bending portion, which produces a load and causes resistance to deteriorate.

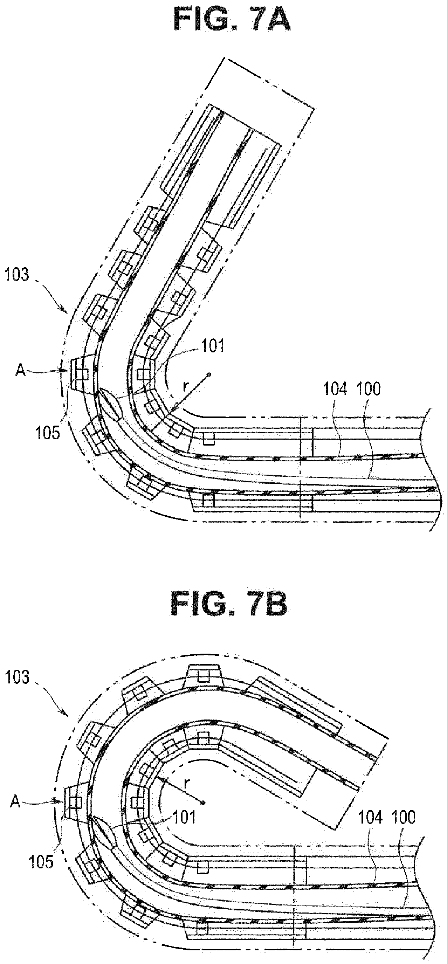

More specifically, as shown in Figs. 7A and 7B, in a conventional bending portion 103, even before a maximum bending state is reached, edges of neighboring bending pieces come into contact with each other and a bending radius r of a plurality of bending pieces bent from a proximal end side becomes smaller just like the bending radius r in the maximum bending state.

Thus, when a treatment instrument 100 is introduced into a treatment instrument channel 104 which is inserted into and disposed in the conventional bending portion 103, if a bending angle is equal to or greater than, for example, 120 degrees as shown in Fig. 7A or although the bending angle of the bending portion 103 is equal to or greater than, for example, 180 degrees as shown in Fig. 7B, in the treatment instrument 100 that moves through the treatment instrument channel 104, a treatment section 101 of the treatment instrument 100 comes into contact with a specific string guide 105 via the treatment instrument channel 104 at substantially the same position (in the vicinity of an intermediate bending piece A in Fig. 7A) in the bending portion 103.

As a result, there is a possibility that resistance of only part of the treatment instrument channel 104 inserted through the conventional bending portion 103 may extremely deteriorate.

If such an operation is repeated in the conventional endoscope, a specific region of the treatment instrument channel in the bending portion is intensively worn or deteriorates, causing the resistance to deteriorate.

The present invention has been implemented in view of the above-described circumstances and it is an object of the present invention to provide an endoscope bending portion and an endoscope that prevent the resistance of a specific region of a treatment instrument channel inserted through and disposed in a bending portion from deteriorating.

### Disclosure of Invention

### Means for Solving the Problems

An endoscope bending portion according to an aspect of the present invention includes a tubular member in which a treatment instrument channel for inserting a treatment instrument is disposed, a plurality of slots are formed in a circumferential direction, and a plurality of string guides that hold a wire on an inner circumferential portion are provided, the tubular member including a first tubular region at a distal end portion, a second tubular region at an intermediate portion and a third tubular region at a proximal end portion, in which a first interval of a plurality of first slots formed in the first tubular region is made to be smaller than a second interval of a plurality of second slots formed in the second region, a third interval of a plurality of third slots formed in the third tubular region is also made to be smaller than the interval of the plurality of second slots, bending rigidity of the second tubular region is made to be higher than bending rigidity of the first tubular region and bending rigidity of the third tubular region, a dimensional ratio between a width of the first slot and the first interval, a dimensional ratio between a width of the second slot and the second interval and a dimensional ratio between a width of the third slot and the third interval are equalized so that an overall radius of curvature corresponding to maximum bending becomes uniform.

An endoscope according to an aspect of the present invention includes an insertion portion that includes an endoscope bending portion including a tubular member in which a treatment instrument channel for inserting a treatment instrument is disposed, a plurality of slots are formed in a circumferential direction, and a plurality of string guides that hold a wire on an inner circumferential portion are provided, the tubular member including a first tubular region at a distal end portion, a second tubular region at an intermediate portion and a third tubular region at a proximal end portion, in which a first interval of a plurality of first slots formed in the first tubular region is made to be smaller than a second interval of a plurality of second slots formed in the second region, a third interval of a plurality of third slots formed in the third tubular region is also made to be smaller than the interval of the plurality of second slots, bending rigidity of the second tubular region is made to be higher than bending rigidity of the first tubular region and bending rigidity of the third tubular region, a dimensional ratio between a width of the first slot and the first interval, a dimensional ratio between a width of the second slot and the second interval and a dimensional ratio between a width of the third slot and the third interval are equalized so that an overall radius of curvature corresponding to maximum bending becomes uniform, and an operation portion connected to the insertion portion and provided with an operation member configured to pull/slacken the wire.

According to the present invention described above, it is possible to provide an endoscope bending portion and an endoscope that prevent the resistance of a specific region of a treatment instrument channel inserted through and disposed in a bending portion from deteriorating.

### Brief Description of the Drawings

Fig. 1 is a perspective view illustrating a configuration of an endoscope according to an aspect of the present invention;
Fig. 2 is a cross-sectional view illustrating a configuration of a distal end portion of an insertion portion of the endoscope according to the aspect of the present invention;
Fig. 3 is a perspective view illustrating a configuration of a bending portion of the insertion portion of the endoscope according to the aspect of the present invention;
Fig. 4 is a cross-sectional view illustrating a configuration of a bending pipe of the endoscope according to the aspect of the present invention;
Fig. 5 is a cross-sectional view illustrating a state in which the bending portion is bent to a predetermined angle in the endoscope according to the aspect of the present invention;
Fig. 6 is a cross-sectional view illustrating a state in which the bending portion is bent to a maximum extent in the endoscope according to the aspect of the present invention;
Fig. 7A is a cross-sectional view illustrating an operation configuration of a bending portion of a conventional insertion portion; and
Fig. 7B is a cross-sectional view illustrating an operation configuration of the bending portion of the conventional insertion portion.

### Best Mode for Carrying Out the Invention

Hereinafter, an endoscope bending portion of an endoscope which is the present invention will be described. Note that in the following description, drawings based on each embodiment are schematic, a relationship between a thickness and a width of each component, a thickness ratio among respective components or the like are different from the real ones, and there may be components dimensional relationships and ratios of which are different among the drawings.

Hereinafter, an endoscope provided with an endoscope insertion portion according to an aspect of the present invention will be described based on the accompanying drawings.

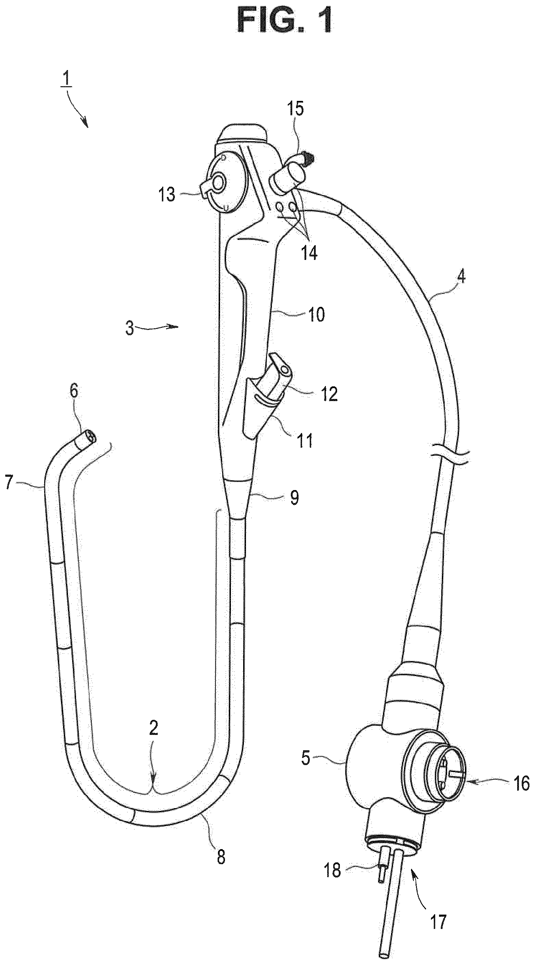

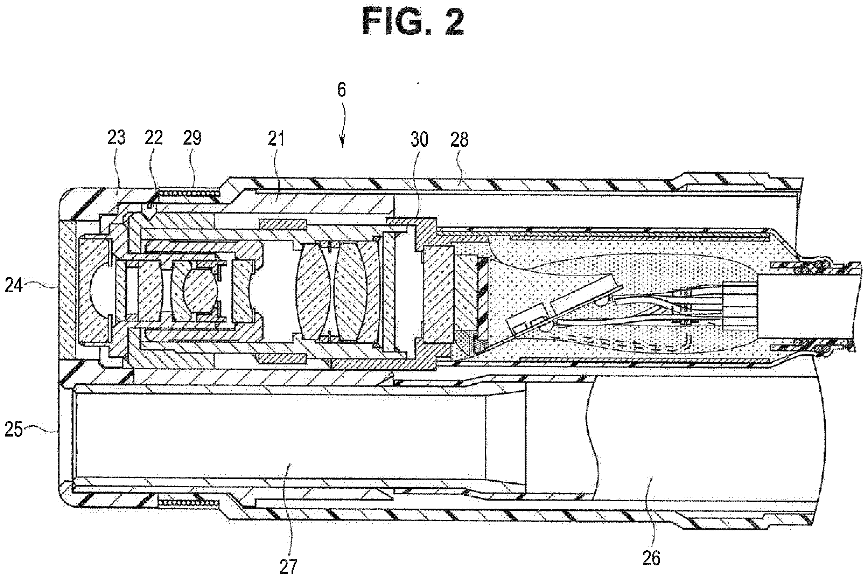

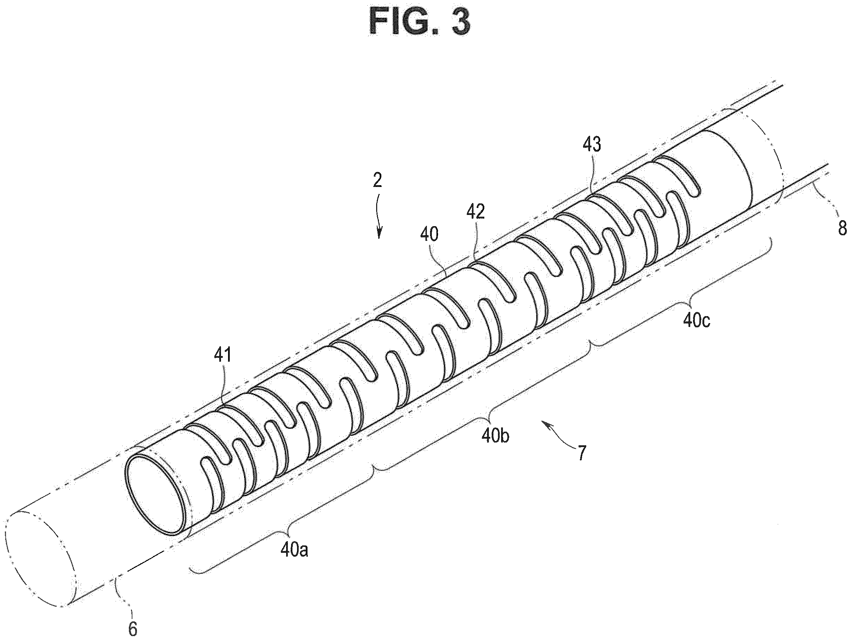

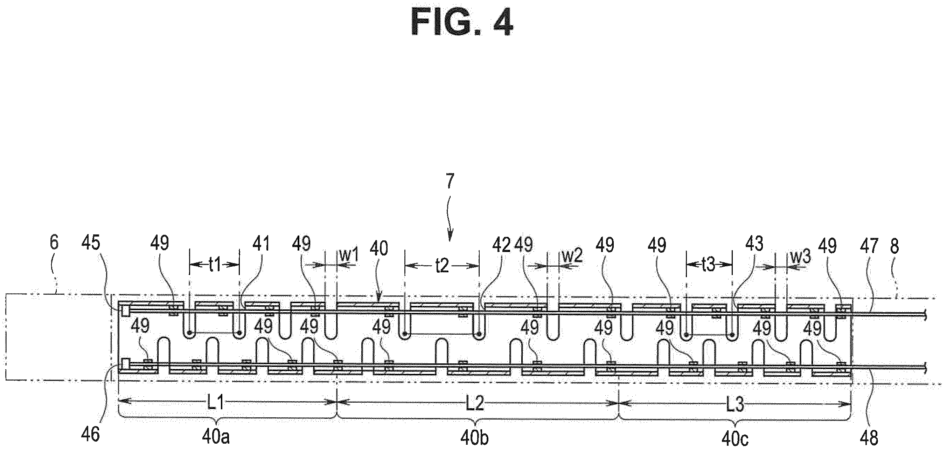

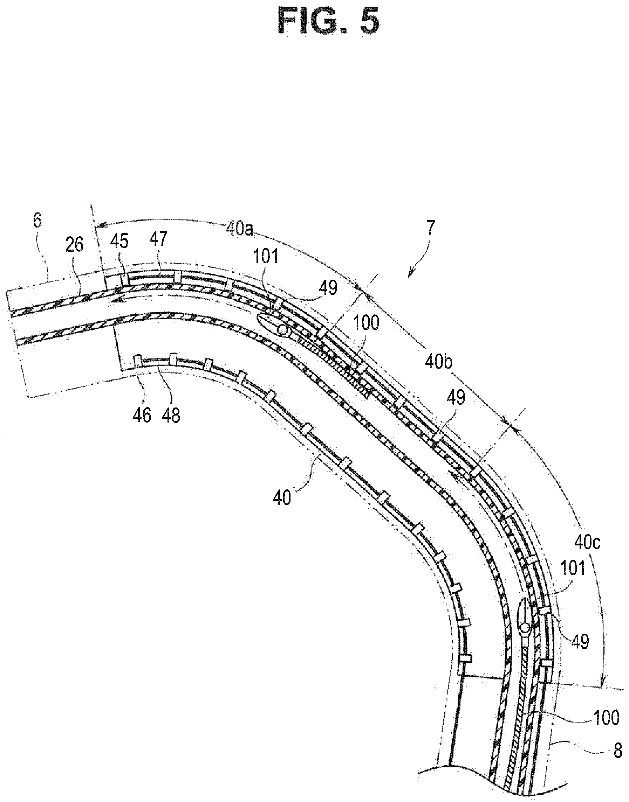

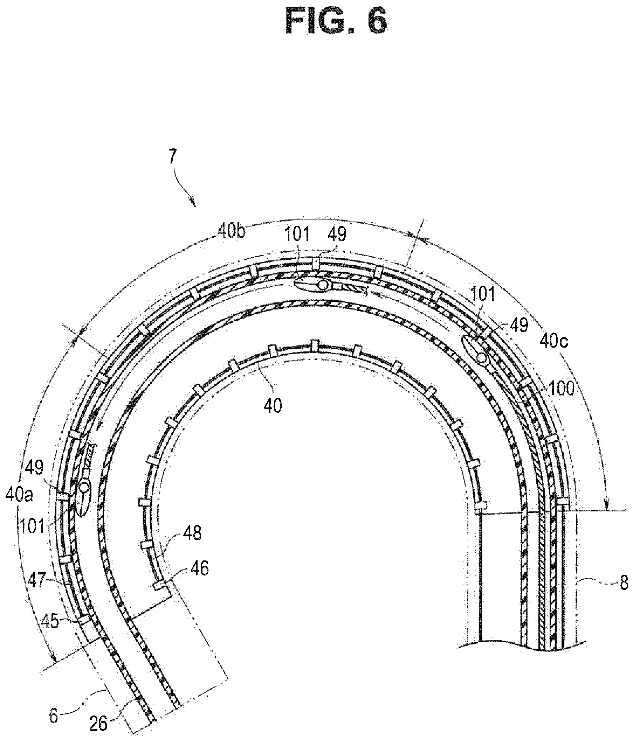

Fig. 1 to Fig. 6 are related to the aspect of the endoscope provided with the endoscope insertion portion of the present invention, Fig. 1 is a perspective view illustrating a configuration of the endoscope, Fig. 2 is a cross-sectional view illustrating a configuration of a distal end portion of the insertion portion, Fig. 3 is a perspective view illustrating a configuration of a bending portion of the insertion portion, Fig. 4 is a cross-sectional view illustrating a configuration of a bending pipe, Fig. 5 is a cross-sectional view illustrating a state in which the bending portion is bent to a predetermined angle, and Fig. 6 is a cross-sectional view illustrating a state in which the bending portion is bent to a maximum extent.

As shown in Fig. 1, an electronic endoscope of the present embodiment (hereinafter simply referred to as "endoscope") 1 is mainly constructed of an insertion portion 2 formed into an elongated tubular shape, an operation portion 3 connected to a proximal end of the insertion portion 2, a universal cord 4 which is an endoscope cable that extends from the operation portion 3, an endoscope connector 5 disposed at a distal end of the universal cord 4, and the like.

The insertion portion 2 is a flexible tubular member formed by connecting a distal end portion 6, a bending portion 7 as an endoscope bending portion of the present embodiment, and a flexible tubular portion 8 in that order from the distal end side. The distal end portion 6 accommodates and arranges an image pickup unit which is an image pickup apparatus, not shown and incorporating image pickup means, illumination means which is not shown, and the like.

Note that the endoscope 1 is not limited to an electronic endoscope and may also be a fiber scope in which an image guide fiber without any image pickup unit is disposed in the insertion portion 2.

The bending portion 7 is a mechanism part configured to be actively bent in two up/down directions (UP-DOWN) through a rotation operation of a bending lever 13 which will be described later among operation members of the operation portion 3.

Note that the bending portion 7 is not limited to this type of bending portion, but may also be of a type that can be bent not only in the up/down directions but also in left/right directions; that is, four directions (all circumferential directions around the axis through up/down, left/right operations; UP-DOWN/RIGHT-LEFT).

The flexible tubular portion 8 is a tubular member formed with flexibility so as to be passively flexible. The flexible tubular portion 8 incorporates not only a treatment instrument insertion channel which will be described later but also various signal lines which will be described later, configured to extend from an image pickup unit incorporated in the distal end portion 6 and further extend from the operation portion 3 into the universal cord 4, a light guide which will be described later, configured to guide illumination light from the light source apparatus and emit the illumination light from the distal end portion 6, and the like (all of which are not shown).

The operation portion 3 is constructed of a bend preventing portion 9 provided on the distal end side, connected to the flexible tubular portion 8 by covering a proximal end of the flexible tubular portion 8, a grasping portion 10 connected to the bend preventing portion 9, and grasped by the user by hand when using the endoscope 1, operation means for operating various endoscope functions provided on an outer surface of the grasping portion 10, a treatment instrument insertion section 11, a suction valve 15, and the like.

Examples of the operation means provided for the operation portion 3 include a bending lever 13 configured to perform bending operation on the bending portion 7, and a plurality of operation members 14 for air/water feeding operation or suction operation, and various operations corresponding to image pickup means and illumination means.

The treatment instrument insertion section 11 is a component provided with a treatment instrument insertion port into which various treatment instruments (not shown) are inserted, communicating with the treatment instrument insertion channel inside the operation portion 3 via a branch member.

The treatment instrument insertion section 11 is provided with a forceps plug 12 which is a cover member to open/close the treatment instrument insertion port and configured to be detachably (replaceably) attached to the treatment instrument insertion section 11.

The universal cord 4 is a composite cable extending from the distal end portion 6 of the insertion portion 2 through the insertion portion 2 to the operation portion 3, through which various signal lines extending from the operation portion 3 are inserted, through which a light guide of a light source apparatus (not shown) is inserted and further through which an air/water feeding tube extending from an air/water feeding apparatus (not shown) is inserted.

The endoscope connector 5 includes an electric connector portion 16 on a side face portion, to which a signal cable connecting itself with a video processor (not shown) which is an external device, a light source connector portion 17 to which a light guide bundle which will be described later configured to connect itself with a light source apparatus which is an external device and an electric cable (not shown) are connected, and an air/water feeding plug 18 to which an air/water feeding tube (not shown) from an air/water feeding apparatus (not shown) which is an external device is connected, or the like.

Next, an internal configuration of the distal end portion 6 of the insertion portion 2 will be described in brief based on Fig. 2.

As shown in Fig. 2, the distal end portion 6 incorporates an image pickup unit 30 which constitutes an image pickup apparatus. The image pickup unit 30 is fitted and disposed into a distal end rigid member 21 which is a rigid distal end portion body, and is firmly fixed to the distal end rigid member 21 together with an adhesive, using a set screw 22 as a fixing member from a lateral direction.

A distal end cover 23 that constitutes a distal end surface of the distal end portion 6 is bonded and fixed so as to cover the distal end portion of the distal end rigid member 21. An observation window 24, an illumination window which is not shown and an observation window cleaning nozzle are hermetically fixed using an adhesive or through screwing.

Note that a distal end opening portion 25 which is a hole portion formed in the distal end cover 23 constitutes an opening portion of the treatment instrument channel 26 in the distal end portion 6. The treatment instrument channel 26 is connected so as to cover a channel connection tube 27, a distal end portion of which is inserted through the distal end rigid member 21.

Furthermore, a bending rubber 28 made of rubber that integrally covers the outer circumference of the distal end rigid member 21 and the bending portion 7 is provided so as to form an exterior shape of the distal end portion 6 and the bending portion 7. A distal end outer circumferential portion of the bending rubber 28 is fixed to the distal end portion 6 using a bobbin adhesion portion 29.

The distal end rigid member 21 is provided not only with the image pickup unit 30 and the channel connection tube 27 but also with a light guide which is not shown, configured to guide illumination light, an observation window cleaning nozzle configured to clean an observation window or the like of the distal end portion 6 or send air into the body cavity and a conduit configured to communicate with a cleaning tube or the like.

Note that since members such as the observation window cleaning nozzle, the cleaning tube and the light guide have conventionally well-known configurations, detailed description of these members will be omitted. Moreover, the image pickup unit 30 also has a conventionally well-known configuration, and so detailed description of the image pickup unit 30 will be omitted.

Here, a configuration of the bending portion 7 provided in the insertion portion 2 of the endoscope 1 of the present embodiment will be described based on Fig. 3 and Fig. 4 hereinafter.

Note that "up" and "down" in the following description coincide with up and down directions in an image of a subject picked up by the image pickup unit 30 and displayed on a monitor or the like and the bending portion 7 is bent in the up and down directions by the first bending lever 13 provided in the operation portion 3 according to the up and down directions.

As shown in Fig. 3 and Fig. 4, the bending portion 7 of the insertion portion 2 is a tubular member and incorporates a bending pipe 40 as the bending tube here. The bending pipe 40 includes a first bending pipe portion 40a at a distal end portion as a first tubular region, a second bending pipe portion 40b at an intermediate portion and a third bending pipe portion 40c at a proximal end.

Note that the bending pipe 40 here is a member mainly composed of a cylindrical ultra-elastic alloy pipe as the bending component. Examples of the ultra-elastic alloy member constituting the bending pipe 40 include Ni-Ti (nickel titanium), titanium alloy, beta titanium, pure titanium, 6-4 titanium and A7075 (aluminum alloy). The bending pipe 40 may also be formed of a resin pipe.

The first bending pipe portion 40a of the bending pipe 40 has a predetermined length of L1 as shown in Fig. 4 and is provided with a plurality of first bending slots 41 having a partially arcuate long hole which extends in a circumferential direction as a basic shape at a predetermined interval (pitch) of t1 through laser processing or the like.

On the other hand, the second bending pipe portion 40b of the bending pipe 40 has a predetermined length of L2 and is provided with a plurality of second bending slots 42 having a partially arcuate long hole which extends in a circumferential direction as a basic shape as in the case of first bending pipe portion 40a at a predetermined interval (pitch) of t2 through laser processing or the like.

Furthermore, the third bending pipe portion 40c of the bending pipe 40 has a predetermined length of L3 and is provided with a plurality of third bending slots 43 having a partially arcuate long hole which extends in a circumferential direction as a basic shape as in the case of first bending pipe portion 40a and the second bending pipe portion 40b at a predetermined interval (pitch) of t3 through laser processing or the like.

The plurality of first bending slots 41, second bending slots 42 and third bending slots 43 are formed at positions up and down alternately in the direction orthogonal to the longitudinal direction of the bending pipe 40.

Note that the bending pipe 40 may be implemented in various combinations of respective predetermined lengths of L1, L2 and L3 of the first bending pipe portion 40a, the second bending pipe portion 40b and the third bending pipe portion 40c; L1, L2 and L3 being the same (L1=L2=L3), being different (L1≠L2≠L3), L1 and L3 being the same (L1=L3) and only L2 being different (L1=L3≠L2) respective relationships of lengths of which is as one example, or the like.

The bending pipe 40 is configured such that the predetermined interval (pitch) t1 of the first bending slots 41 formed in the first bending pipe portion 40a and the predetermined interval (pitch) t3 of the third bending slots 43 formed in the third bending pipe portion 40c are set to be smaller (shorter, t1<t2, t3<t2) than the predetermined interval (pitch) t2 of the second bending slots 42 formed in the second bending pipe portion 40b.

Thus, the bending pipe 40 is configured such that the first bending pipe portion 40a on the distal end side and the third bending pipe portion 40c on the proximal end side are set to have lower bending rigidity than the second bending pipe portion 40b at the intermediate portion.

That is, the bending portion 7 of the insertion portion 2 according to the present embodiment is configured to have lower bending rigidity (softer) on the distal end side and on the proximal end side than at the intermediate portion.

Furthermore, regarding the predetermined interval (pitch) t1 of the first bending slots 41 and the predetermined interval (pitch) t3 of the third bending slots 43, the predetermined interval (pitch) t1 is preferably set to be smaller (shorter, t1<t3) than the predetermined interval (pitch) t3 and the first bending pipe portion 40a on the distal end side preferably has lower bending rigidity than the third bending pipe portion 40c on the proximal end side.

Note that the predetermined interval (pitch) t1 of the first bending slots 41 and the predetermined interval (pitch) t3 of the third bending slots 43 may have the same predetermined interval (t1=t3) and the first bending pipe portion 40a on the distal end side and the third bending pipe portion 40c on the proximal end side may have the same bending rigidity.

Furthermore, the bending pipe 40 provided in the bending portion 7, when bent to a maximum extent, is configured such that the first bending pipe portion 40a, the second bending pipe portion 40b and the third bending pipe portion 40c have the same radius of curvature. That is, when the bending portion 7 of the present embodiment is bent to a maximum extent, the whole bending pipe 40 is bent at a uniform radius of curvature.

More specifically, the bending portion 7 is configured such that a dimensional ratio of length between a slot width w1 of the first bending slots 41 formed in the first bending pipe portion 40a of the bending pipe 40 provided inside the bending portion 7 and a predetermined interval (pitch) t1 of the first bending slots 41 (w1:t1), a dimensional ratio of length between a slot width w2 of the second bending slots 42 formed in the second bending pipe portion 40b and a predetermined interval (pitch) t2 of the second bending slots 42 (w2:t2) and a dimensional ratio of length between a slot width w3 of the third bending slots 43 formed in the third bending pipe portion 40c and a predetermined interval (pitch) t3 of the third bending slots 43 (w3:t3) are set to be equal (w1:t1=w2:t2=w3:t3).

Thus, when the bending portion 7 is bent to a maximum extent, all the first bending pipe portion 40a, the second bending pipe portion 40b and the third bending pipe portion 40c of the bending pipe 40 inside the bending portion 7 have bending shapes with the same radius of curvature, and the whole bending pipe 40 is bent at a uniform radius of curvature accordingly.

Note that regarding the first bending pipe portion 40a, the second bending pipe portion 40b and the third bending pipe portion 40c, the respective bending slots 41, 42 and 43, and the predetermined intervals (pitches) t1, t2 and t3 of the respective bending slots 41, 42 and 43 may be freely set in various ways at the manufacturing stage so that the respective bending pipe portions have different desired radii of curvature when bent to a maximum extent and the whole bending pipe may be bend at a non-uniform radius of curvature.

The bending pipe 40 configured as described above is provided with a pair of angle wires 47 and 48 for bending in the up/down direction connected to any one of two wire stoppers 45 and 46 provided up and down in an inner circumferential portion of the distal end portion of the first bending pipe portion 40a.

The pair of angle wires 47 and 48 are inserted through and held by a plurality of string guides 49 provided in the up and down inner circumferential portions of the respective bending pipe portions 40a, 40b and 40c of the bending pipe 40.

The pair of angle wires 47 and 48 are disposed inside the insertion portion 2, inserted up to the operation portion 3 and pulled/slackened by the bending lever 13. The bending portion 7 is bent when the pair of angle wires 47 and 48 are mutually pulled/slackened according to the operation of the bending lever 13.

That is, the bending portion 7 provided in the insertion portion 2 of the endoscope 1 is bent when the pair of angle wires 47 and 48 are mutually pulled/slackened through hand operation of the bending lever 13.

During the bending operation, since the first bending pipe portion 40a on the distal end side of the bending pipe 40 and the third bending pipe portion 40c on the proximal end side have lower rigidity than the second bending pipe portion 40b at the intermediate portion, until the bending portion 7 reaches a predetermined bending angle from the linear state, the endoscope 1 is placed in a state in which the distal end portion and the proximal end portion are bent first as shown in, for example, Fig. 5.

At this time, due to rigidity of the second bending pipe portion 40b, the bending portion 7 is hardly bendable up to a predetermined bending angle, and so the bending portion 7 remains substantially linear with the intermediate portion substantially not being bent.

When the treatment instrument 100 is inserted through the treatment instrument channel 26 in this condition, a force of the treatment section 101 of the treatment instrument 100 that presses the treatment instrument channel 26 increases when the treatment instrument 100 passes through the bending part to an outward side at the first bending pipe portion 40a on the distal end side and the third bending pipe portion 40c on the proximal end side which are bent.

For this reason, when the treatment section 101 of the treatment instrument 100 inserted into the treatment instrument channel 26 passes, the outer circumferential portions located at the first bending pipe portion 40a and the third bending pipe portion 40c receive a large load whereby the outer circumferential portions are pressed against the string guide 49.

Note that since the second bending pipe portion 40b is substantially linear, the portion of the treatment instrument channel 26 located at the second bending pipe portion 40b receives a load whereby the portion is pressed against the string guide 49, which is significantly smaller than the load on the first bending pipe portion 40a and the third bending pipe portion 40c.

When the bending portion 7 is bent from the predetermined angle shown in Fig. 5 to a maximum extent as shown in Fig. 6, the second bending pipe portion 40b at the intermediate portion is also bent together with the first bending pipe portion 40a on the distal end side of the bending pipe 40 and the third bending pipe portion 40c on the proximal end side.

Thus, in the process of bending from the predetermined angle to the maximum extent of the bending portion 7, since the rigidity of the second bending pipe portion 40b at the intermediate portion of the bending pipe 40 is higher than those of the first bending pipe portion 40a on the distal end side and the third bending pipe portion 40c on the proximal end side, the intermediate portion is bent with a greater radius of curvature than those of the distal end portion and the proximal end portion.

Note that Fig. 6 illustrates a configuration in which when the bending portion 7 is bent to the maximum extent, the whole bending pipe portion is bent at a uniform radius of curvature.

When the treatment instrument 100 is inserted through the treatment instrument channel 26 while the bending portion 7 is bent to the maximum extent, the force of the treatment section 101 of the treatment instrument 100 that presses the treatment instrument channel 26 against the bending outward side is strongest at the second bending pipe portion 40b at the intermediate portion on the distal end side of the bending pipe 40 when the treatment section 101 passes.

For that reason, when the treatment section 101 of the treatment instrument 100 inserted through the treatment instrument channel 26 passes, the outer circumferential portion located at the second bending pipe portion 40b is pressed against the string guide 49 provided on the bending outward side more than the first bending pipe portion 40a and the third bending pipe portion 40c, and the load increases.

Thus, in the endoscope 1 of the present embodiment, when the bending portion 7 provided in the insertion portion 2 is bent from a linear state to a maximum extent, only the distal end portion and the proximal end portion change so as to substantially bend until the bending angle reaches a predetermined angle and the intermediate portion changes so as to bend from an angle equal to or greater than the predetermined angle.

By adopting such a configuration, when the treatment instrument 100 is inserted through the treatment instrument channel 26 in the bending portion 7 of the endoscope 1 while the bending portion 7 is bent, since the portion of the treatment instrument channel 26 where a large load is produced when the treatment instrument channel 26 is pressed against the string guide 49, that is, the portion of the treatment instrument channel 26 where wear, deterioration or the like is likely to occur, changes, it is possible to reduce intensive wearing, deterioration or the like of a specific region of the treatment instrument channel 26 and prevent deterioration in resistance of the treatment instrument channel 26 compared to the prior art.

According to the description above, the endoscope 1 of the present embodiment can be configured to disperse the load on the treatment instrument channel 26 that presses it against the string guide 49 when the treatment instrument 100 is inserted through the treatment instrument channel 26 in the bending portion 7 and prevent deterioration in resistance of the treatment instrument channel 26.

That is, the endoscope 1 can prevent deterioration in resistance of a specific region of the treatment instrument channel 26 inserted through and disposed in the bending portion 7.

Note that the invention described in the above embodiment is not limited to the aforementioned embodiment and modification, and in addition, various modifications can be made without departing from the spirit and scope of the present invention in the implementation stage. Furthermore, the above embodiment includes inventions in various stages and various kinds of invention can be extracted with an appropriate combination of a plurality of configuration requirements disclosed.

For example, when several configuration requirements are deleted from all configuration requirements disclosed in the above embodiment, if the mentioned problems to be solved by the invention can be solved and advantageous effects of the invention can be achieved, the configuration from which the configuration requirements are deleted can be extracted as an invention.

The present application claims priority based on Japanese Patent Application No. 2015-019689, filed in Japan on February 3, 2015, the disclosure of which is incorporated in the specification, claims and drawings of the present application by reference.

## Claims

1. An endoscope bending portion comprising a tubular member in which a treatment instrument channel for inserting a treatment instrument is disposed, a plurality of slots are formed in a circumferential direction, and a plurality of string guides that hold a wire on an inner circumferential portion are provided, the tubular member comprising a first tubular region, a second tubular region and a third tubular region provided at a distal end portion, an intermediate portion and a proximal end portion respectively,
wherein a first interval of a plurality of first slots formed in the first tubular region is made to be smaller than a second interval of a plurality of second slots formed in the second region, a third interval of a plurality of third slots formed in the third tubular region is also made to be smaller than the interval of the plurality of second slots, bending rigidity of the second tubular region is made to be higher than bending rigidity of the first tubular region and bending rigidity of the third tubular region, and
a dimensional ratio between a width of the first slot and the first interval, a dimensional ratio between a width of the second slot and the second interval and a dimensional ratio between a width of the third slot and the third interval are equalized so that an overall radius of curvature corresponding to maximum bending becomes uniform.

2. The endoscope bending portion according to claim 1, wherein the plurality of slots are formed at positions up and down alternately in a direction orthogonal to a longitudinal direction of the tubular member.

3. The endoscope bending portion according to claim 1 or 2, wherein the first interval is smaller than the third interval.

4. An endoscope comprising:
an insertion portion comprising the endoscope bending portion according to any one of claims 1 to 3; and
an operation portion connected to the insertion portion and provided with an operation member configured to pull/slacken the wire.
